# EUROPÄISCHE PATENTANMELDUNG

(11) **EP 4 442 193 A1**
(43) Veröffentlichungstag der Anmeldung: **09.10.2024**
(21) Anmeldenummer: 23166330.3
(22) Anmeldetag: 03.04.2023
(51) Int. Cl.: A61B 5/00, G01R 33/36, G01R 33/28, G01R 33/567

(54) **OBJEKTTISCH FÜR EIN MAGNETRESONANZGERÄT**

(71) Anmelder: Siemens Healthineers AG, 91301 Forchheim (DE)
(72) Erfinder: Biber, Stephan, 91056 Erlangen (DE); Bollenbeck, Jan, 91330 Eggolsheim (DE); Höcht, Philipp, 91207 Lauf (DE); Meise, Florian, 91353 Hausen (DE)
(74) Vertreter: Siemens Healthineers Patent Attorneys

(57) **Zusammenfassung**

Die Erfindung betrifft einen Objekttisch (26) für ein bildgebendes Untersuchungsgerät (14), das zum Durchführen einer Untersuchung an einem auf dem Objekttisch (26) anordbaren Objekt (12) dient, wobei der Objekttisch (26) ausgebildet ist, an einem Rand eines Erfassungsbereichs (22) des bildgebenden Untersuchungsgeräts (14) angeordnet zu werden, um das zu untersuchende Objekt (12) zum Durchführen der Untersuchung im Erfassungsbereich (22) anzuordnen, wobei der Objekttisch (26) an einem Tischrand (60) zumindest bereichsweise eine entlang des Tischrands (60) verlaufende Verbindungseinheit (64) aufweist, die ausgebildet ist, mit einem Verbindungselement (66) wenigstens eines für die Untersuchung verwendbaren Zubehörteils (68) lösbar verbunden zu werden.

Erfindungsgemäß weist die Verbindungseinheit (64) eine Koppeleinheit (70) auf, wobei die Koppeleinheit ausgebildet ist, in einem verbundenen Zustand, in dem die Verbindungseinheit (64) mit dem Verbindungselement (66) verbunden ist, mit dem Zubehörteil (68) eine Koppelanordnung (72) auszubilden, wobei die Koppelanordnung (72) einem Übertragen von zumindest Daten oder Energie dient.

## Beschreibung

Die Erfindung betrifft einen Objekttisch für ein bildgebendes Untersuchungsgerät, das zum Durchführen einer Untersuchung an einem auf dem Objekttisch anordbaren Objekt dient, wobei der Objekttisch ausgebildet ist, an einem Rand eines Erfassungsbereichs des bildgebendes Untersuchungsgeräts angeordnet zu werden, um das zu untersuchende Objekt zum Durchführen der Untersuchung im Erfassungsbereich anzuordnen, wobei der Objekttisch an einem Tischrand zumindest bereichsweise eine entlang des Tischrands verlaufende Verbindungseinheit aufweist, die ausgebildet ist, mit einem Verbindungselement wenigstens eines für die Untersuchung verwendbaren Zubehörteils lösbar verbunden zu werden. Die Erfindung betrifft ferner ein bildgebendes Untersuchungsgerät zum Durchführen einer Untersuchung an einem Objekt, mit einem Objekttisch zum Anordnen des Objekts sowie wenigstens einem Zubehörteil.

Bildgebende Untersuchungsgeräte, wie zum Beispiel Magnetresonanzgeräte, Computertomographen, oder dergleichen, sowie Objekttische für bildgebende Untersuchungsgeräte sind im Stand der Technik umfänglich bekannt, sodass es eines gesonderten druckschriftlichen Nachweises hierfür dem Grunde nach nicht bedarf. Gattungsgemäße Vorrichtungen dienen dazu, am Objekt eine Untersuchung durchzuführen, um eine Struktur des Objekts zumindest teilweise zu ermitteln. Solche Untersuchungen werden häufig in der Materialprüfung, aber auch im Bereich einer Diagnoseerstellung bei biologischem Material, Lebewesen oder dergleichen eingesetzt. Das Objekt kann demnach zum Beispiel ein Gegenstand als Ergebnis eines industriellen Fertigungsprozesses, aber auch in Abbauprodukt aus dem Bergbau, ein Körper eines Lebewesens oder dergleichen sein.

In der Medizintechnik zeichnet sich die Bildgebung mittels Magnetresonanz (MR), auch Magnetresonanztomographie (MRT; Englisch: magnetic resonance imaging, MRI) genannt, durch hohe Kontraste besonders bei Weichteilen aus.

Zur Durchführung der Untersuchung werden mittels einer Magnetfeldquelle Magnetfeldimpulse erzeugt und das Objekt hiermit beaufschlagt. Das Magnetfeld kann magnetische Flussdichten im Bereich von einigen Tesla aufweisen. Aufgrund von Wechselwirkungen des Objekts mit dem Magnetfeld auf atomarer Ebene können in dem Objekt so genannte MR-Signale ausgelöst werden, indem insbesondere zunächst Protonenspins ausgerichtet werden, wobei aufgrund der Einwirkung von hochfrequenten Magnetimpulsen die MR-Signale erzeugt werden. Die MR-Signale können mit einer Lokalspule, die vorzugsweise im Untersuchungsbereich des Objekts angeordnet ist, beispielsweise an einer Oberfläche des Objekts, detektiert und mittels einer Auswerteeinheit des Magnetresonanzgeräts ausgewertet werden. Die Auswerteeinheit kann Bestandteil eines Magnetresonanzgeräts sein oder als separate Einheit mit dem Magnetresonanzgerät und/oder der Lokalspule verbunden sein. Als Ergebnis der Auswertung kann zum Beispiel eine Bildgebung in Bezug auf die Struktur des Objekts vorgesehen sein.

Die während des bestimmungsgemäßen Betriebs, das heißt, insbesondere während einer Durchführung einer Untersuchung am Objekt, auftretenden magnetischen Feldstärken haben in der Regel sehr große Werte der magnetischen Flussdichte zur Folge, beispielsweise in einem Bereich von etwa 1T bis etwa 7T oder dergleichen. Die große magnetische Flussdichte kann auch außerhalb des Erfassungsbereichs des Magnetresonanzgeräts noch wirksam sein und hier andere Geräte, beispielsweise ein Zubehörteil, beeinflussen. Ein Zubehörteil kann zum Beispiel eine Tasche, eine Wanne oder dergleichen sein, welches bedarfsweise zur Durchführung der Untersuchung genutzt werden kann. Darüber hinaus kann das Zubehörteil auch ein Elektrokardiograph (EKG), ein Blutdruckmessgerät und/oder dergleichen sein. Je nach Bedarf können auch weitere beziehungsweise mehrere Zubehörteile vorgesehen sein.

Das Zubehörteil ist vorzugsweise in dem Raum angeordnet, in dem auch das bildgebende Untersuchungsgerät aufgestellt ist. Dadurch ist es für die Durchführung der Untersuchung vorzunehmendes Personal leicht, bei Bedarf auf das jeweilige Zubehörteil zuzugreifen. Besonders elektrische und/oder elektronische Zubehörteile, die beispielsweise drahtlos im bestimmungsgemäßen Betrieb genutzt werden können, benötigen zumindest zeitweise eine Versorgung mit elektrischer Energie und/oder eine kommunikationstechnische Verbindung zu einem Kommunikationsnetzwerk, beispielsweise einer externen Auswerteeinheit, einer Zentrale und/oder dergleichen. Häufig stehen diese Funktionen innerhalb des Raums, in dem das bildgebende Untersuchungsgerät angeordnet ist, nicht ohne weiteres zur Verfügung. Daher sind derartige Zubehörteile zumindest zeitweise außerhalb des Bereichs des bildgebenden Untersuchungsgeräts, insbesondere außerhalb des Untersuchungsraums, in dem das bildgebende Untersuchungsgerät aufgestellt ist, angeordnet. Dies ist jedoch für die Nutzung des bildgebenden Untersuchungsgeräts ungünstig.

Der Erfindung liegt die Aufgabe zugrunde, eine Aufbewahrungsmöglichkeit für elektrische beziehungsweise elektronische Zubehörteile bereitzustellen, die die vorgenannten Probleme vermeidet und eine Anordnung im Bereich des bildgebenden Untersuchungsgeräts ermöglicht.

Als Lösung werden mit der Erfindung ein Objekttisch und ein bildgebendes Untersuchungsgerät gemäß den unabhängigen Ansprüchen vorgeschlagen.

Vorteilhafte Weiterbildungen ergeben sich durch Merkmale der abhängigen Ansprüche.

In Bezug auf einen gattungsgemäßen Objekttisch wird mit der Erfindung insbesondere vorgeschlagen, dass die Verbindungseinheit eine Koppeleinheit aufweist, wobei die Koppeleinheit ausgebildet ist, in einem Anordnungszustand, in dem die Verbindungseinheit mit dem Verbindungselement verbunden ist, mit dem Zubehörteil eine Koppelanordnung auszubilden, wobei die Koppelanordnung einem Übertragen von zumindest Daten oder Energie dient.

In Bezug auf ein gattungsgemäßes bildgebendes Untersuchungsgerät wird mit der Erfindung insbesondere vorgeschlagen, dass der Objekttisch gemäß der Erfindung ausgebildet ist.

Die Erfindung basiert unter anderem auf dem Gedanken, dass die Verbindungseinheit mit einer Koppeleinheit kombiniert werden kann, die es erlaubt, eine kommunikationstechnische und/oder energietechnische Kopplung zum Zubehörteil zu realisieren. Das Zubehörteil ist diesbezüglich entsprechend angepasst ausgebildet. Das Zubehörteil kann ein entsprechendes Koppelmodul aufweisen, welches ausgebildet ist, mit der Koppeleinheit eine energietechnische und/oder kommunikationstechnische Kopplung auszubilden. Dadurch kann die Koppelanordnung hergestellt werden.

Das Zubehörteil kann ein bereits vorhandenes Zubehörteil sein, welches mit einer separaten Ladestation oder Kommunikationsstation entsprechend gekoppelt werden kann. Dadurch dass die Verbindungseinheit die Koppeleinheit bereitzustellen vermag, kann das Zubehörteil im Bereich des bildgebenden Untersuchungsgeräts, nämlich am Objekttisch lösbar angeordnet werden, wobei zugleich eine kommunikationstechnische und/oder energietechnische Kopplung realisiert werden kann. Es ist daher nicht mehr erforderlich, das Zubehörteil außerhalb des Raums, in dem das bildgebende Untersuchungsgerät angeordnet ist, mit einer entsprechenden Station zu koppeln.

Die Koppelanordnung kann ausgebildet sein, eine leitungsgebundene Kopplung zu realisieren. Hierzu kann vorgesehen sein, dass die Koppeleinheit, sowie auch das Koppelmodul des Zubehörteils entsprechende elektrische Kontakte aufweist, die lösbar miteinander verbunden werden können, wenn das Zubehörteil mit seinem Verbindungselement mit der Verbindungseinheit mechanisch verbunden ist. Über die Koppelanordnung kann somit eine energietechnische Kopplung hergestellt werden, die es erlaubt, das Zubehörteil mit elektrischer Energie zu versorgen, insbesondere wenn das Zubehörteil für seinen bestimmungsgemäßen Betrieb einen elektrischen Energiespeicher aufweist. Es ist somit möglich, den Energiespeicher des Zubehörteils mit elektrischer Energie aufzuladen, sodass das Zubehörteil nahezu jederzeit betriebsbereit ist und bedarfsweise zum Zweck der Untersuchung eingesetzt werden kann. Das gleiche gilt dem Grunde nach auch, wenn die Koppelanordnung alternativ oder ergänzend eine kommunikationstechnische Kopplung bilden kann. Es ist dann möglich, über die kommunikationstechnische Kopplung beispielsweise Daten, die das Zubehörteil gespeichert hat, abzurufen und einer Auswerteeinheit oder einer Zentrale für die weitere Datenverarbeitung oder Speicherung zuzuführen. Darüber hinaus ist es auch möglich, dem Zubehörteil über die Koppelanordnung Daten für den bestimmungsgemäßen Betrieb zukommen zu lassen, beispielsweise wenn das Zubehörteil eine programmgesteuerte Rechnereinheit oder dergleichen aufweist. Darüber hinaus können auch Betriebsdaten, beispielsweise Parameter oder dergleichen, an das Zubehörteil übertragen werden. Dadurch ist es möglich, das Zubehörteil im am Objekttisch angeordneten Zustand hinsichtlich seines bestimmungsgemäßen Betriebs aktualisiert zu halten.

Insgesamt erlaubt es die Erfindung, die Nutzung des Zubehörteils weiter zu verbessern. Dabei kann zugleich erreicht werden, dass das Zubehörteil im Bereich des Objekttischs beziehungsweise des bildgebenden Untersuchungsgeräts gelagert werden kann, sodass es bei Bedarf nahezu unverzüglich genutzt werden kann, ohne dass ein Suchen des Zubehörteils durch das Personal oder dergleichen erforderlich wäre. Darüber hinaus kann erreicht werden, dass das Zubehörteil betriebsnah gelagert werden kann. Darüber hinaus ist es möglich, das Zubehörteil an vorgebbaren Stellen anzuordnen, sodass es möglichst schnell verfügbar ist, beispielsweise in Notsituationen oder dergleichen.

Das Zubehörteil kann darüber hinaus geschützt angeordnet werden, sodass es während der bestimmungsgemäßen Durchführung einer Untersuchung mit dem bildgebenden Untersuchungsgerät möglichst wenig beeinträchtigt wird. Obwohl das Zubehörteil in der Nähe des bildgebenden Untersuchungsgeräts angeordnet sein kann, kann erreicht werden, dass der Betrieb des bildgebenden Untersuchungsgeräts im Wesentlichen nicht gestört ist. Umgekehrt kann erreicht werden, dass der bestimmungsgemäße Betrieb des bildgebenden Untersuchungsgeräts das Zubehörteil im Wesentlichen nicht stört.

Die Erfindung ist aber nicht auf die Anwendung eines einzigen Zubehörteils beschränkt. Natürlich können mehrere Zubehörteile vorgesehen sein, die entsprechend am Objekttisch angeordnet werden können. Es kann vorgesehen sein, dass für jedes Zubehörteil eine eigene Verbindungseinheit mit einer jeweiligen Koppeleinheit vorgesehen ist. Darüber hinaus kann aber auch vorgesehen sein, dass die Verbindungseinheit ausgebildet ist, mit mehreren Zubehörteilen beziehungsweise deren Verbindungselementen lösbar verbunden zu werden. Vorzugsweise ist die Koppeleinheit für den Betrieb von mehreren Zubehörteilen ausgebildet.

Die Verbindungseinheit kann mechanische Verbindungsbauteile aufweisen, die es erlauben, eine lösbare Verbindung mit dem Verbindungselement des Zubehörteils auszubilden. Das Verbindungselement kann entsprechend der Verbindungseinheit angepasst ausgebildet sein. Beispielsweise kann die Verbindungseinheit einen oder mehrere Haken oder Ösen aufweisen, die mit entsprechenden Gegenelementen des Verbindungselements verbunden werden können. Darüber hinaus ist es natürlich auch möglich, dass die Verbindungseinheit als kontinuierlich verlaufende Verbindungseinheit entlang des Tischrands ausgebildet ist, beispielsweise nach Art einer Nut oder dergleichen. Die Nut kann zum Beispiel als hinterschnittene Nut, beispielsweise nach Art einer T-Nut, einer Schwalbenschwanznut und/oder dergleichen, ausgebildet sein. In diesem Fall kann vorgesehen sein, dass die Nut an einem Ende offen ausgebildet ist, damit ein entsprechend ausgebildetes Verbindungselement, beispielsweise nach Art eines Pilzkopfelements oder dergleichen in die Nut eingeführt werden kann.

Es kann aber auch vorgesehen sein, dass das Verbindungselement derart ausgestaltet ist, dass es an einer vorgegebenen Position der Verbindungseinheit lösbar mit der Verbindungseinheit verbunden werden kann. Zu diesem Zweck kann zum Beispiel das Verbindungselement bewegbar ausgebildet sein, sodass es eine entsprechende Kontur der Verbindungseinheit hintergreifen kann, sodass es in dieser festgelegt werden kann, beispielsweise nach Art eines Bajonettverschlusses oder dergleichen.

Mit dem Verbinden des Verbindungselements mit der Verbindungseinheit wird die Koppelanordnung ausgebildet, sodass das Übertragen von Daten und/oder Energie erreicht werden kann. Das Verbindungselement kann entsprechend ausgebildet sein. Es kann aber auch vorgesehen sein, dass die Koppelanordnung mittels des Koppelmoduls des Zubehörteils realisiert wird. Natürlich können auch Kombinationen hiervon vorgesehen sein. Die Koppelanordnung braucht nicht für sämtliche Zubehörteile gleich in Bezug auf die Funktionalität ausgebildet sein. Die Verbindungseinheit kann eine Koppeleinheit aufweisen, die für unterschiedliche Kopplungsmöglichkeiten beziehungsweise Kopplungsanordnungen ausgebildet ist.

Der Objekttisch ist vorzugsweise eine Vorrichtung, die es erlaubt, das Objekt anordnen zu können und zum Zwecke der Untersuchung in den Erfassungsbereich einzuführen oder auszuführen. Der Erfassungsbereich, gelegentlich auch Scannerbereich genannt, kann zum Beispiel durch eine Durchgangsöffnung des bildgebenden Untersuchungsgeräts gebildet sein, die axial endseitig jeweilige Mündungen aufweist. Die Mündung kann dann einen jeweiligen Rand bereitstellen. Der Erfassungsbereich kann aber auch zum Beispiel durch einen offenen C-Boden, beispielsweise Gantry, oder dergleichen gebildet sein. Der C-Bogen kann dann beispielsweise den Rand bereitstellen. Der Objekttisch kann daher dazu dienen, das Objekt insbesondere bei einer Durchgangsöffnung als Erfassungsbereich zum Zwecke der Untersuchung durch die Mündung der Durchgangsöffnung des bildgebenden Untersuchungsgeräts in die Durchgangsöffnung einzuführen.

Zum Zwecke des Einführens des Objekts in den Erfassungsbereich kann der Objekttisch vorzugsweise eine entsprechend geeignet ausgebildete und verfahrbare Objektauflage aufweisen, die in den Erfassungsbereich, insbesondere die Durchgangsöffnung, verfahren werden kann. Der Objekttisch kann fest im Bereich des Randes des Erfassungsbereichs, insbesondere der Mündung der Durchgangsöffnung, am bildgebenden Untersuchungsgerät angeordnet sein. Darüber hinaus kann natürlich auch vorgesehen sein, dass der Objekttisch verfahrbar ausgebildet ist und bedarfsgerecht am Rand des Erfassungsbereichs, insbesondere im Bereich der Mündung der Durchgangsöffnung, angeordnet werden kann, sodass das auf dem Objekttisch angeordnete Objekt in den Erfassungsbereich beziehungsweise in die Durchgangsöffnung eingeführt und/oder wieder aus dem Erfassungsbereich beziehungsweise der Durchgangsöffnung herausgeführt werden kann. Das Objekt kann auf der Objektauflage angeordnet werden. Vorzugsweise kann das Objekt im Wesentlichen aufgrund einer Gewichtkraft auf der Objektauflage angeordnet beziehungsweise positioniert bleiben.

Die Koppeleinheit kann mit einer elektrischen Energiequelle und/oder einer Kommunikationseinheit elektrisch verbunden sein. Die elektrische Energiequelle und/oder die Kommunikationseinheit können im Objekttisch selbst angeordnet sein. Natürlich können sie zumindest teilweise auch im bildgebenden Untersuchungsgerät oder sogar extern hiervon angeordnet sein. Die Koppeleinheit steht mit der Energiequelle und/oder der Kommunikationseinheit in Verbindung, sodass die gewünschte Funktionalität in Bezug auf das Übertragen von Energie und/oder Daten erreicht werden kann.

Die Energiequelle kann zum Beispiel durch ein Energieversorgungsnetz, insbesondere einem öffentlichen Energieversorgungsnetz gebildet sein.

Die Kommunikationseinheit kann eine Kommunikationsverbindung zu einem Kommunikationsnetzwerk, insbesondere dem Internet, bereitstellen.

Gemäß einer Weiterbildung wird vorgeschlagen, dass die Koppeleinheit als elektrische Koppeleinheit zum leitungsgebundenen oder drahtlosen elektrischen Koppeln des Zubehörteils ausgebildet ist. Dadurch ist es möglich, eine energietechnische Kopplung zwischen der Verbindungseinheit und dem Zubehörteil herzustellen. Ist ein leitungsgebundenes elektrisches Koppeln vorgesehen, kann vorgesehen sein, dass die Koppeleinheit zum Beispiel Stromschienen aufweist, beispielsweise zwei Stromschienen oder mehrere Stromschienen, die von elektrischen entsprechenden Kontakten des Koppelmoduls beziehungsweise Verbindungselements des Zubehörteils kontaktiert werden können. Die elektrischen Kontakte können beispielsweise in dem Verbindungselement des Zubehörteils integriert ausgebildet sein. Mit dem Verbinden des Verbindungselements mit der Verbindungseinheit kann eine elektrische Verbindung durch entsprechende Kontakte des Koppelmoduls beziehungsweise des Verbindungselements mit den Stromschienen realisiert werden, um die Koppelanordnung nach Art einer elektrischen Kopplung zu realisieren. Alternativ oder zusätzlich kann vorgesehen sein, dass die elektrische Koppeleinheit zum drahtlosen elektrischen Koppeln des Zubehörteils ausgebildet ist. In diesem Fall kann die Nutzung eines Energiefeldes zum drahtlosen elektrischen Koppeln vorgesehen sein.

Gemäß einer weiteren Ausgestaltung wird vorgeschlagen, dass die Koppeleinheit als kommunikationstechnische Koppeleinheit zum leitungsgebundenen oder drahtlosen kommunikationstechnischen Koppeln des Zubehörteils ausgebildet ist. Diese Ausgestaltung kann natürlich auch mit der zuvor erläuterten Weiterbildung in Bezug auf das leitungsgebundene oder drahtlose elektrische Koppeln des Zubehörteils zum Zwecke der Energieübertragung kombiniert sein. Die Koppeleinheit kann auch hier als leitungsgebundene Koppeleinheit ausgebildet sein und zum kommunikationstechnischen Koppeln entsprechende Kommunikationsleitungen aufweisen, die durch das entsprechend ausgebildete Koppelmodul des Zubehörteils lösbar kontaktiert werden können. Dadurch kann eine leitungsgebundene Kopplung realisiert werden. Das kommunikationstechnische Koppeln kann aber auch drahtlos realisiert sein, beispielsweise unter Nutzung von elektrischen und/oder magnetischen Feldern, Licht, insbesondere Infrarotlicht, und/oder dergleichen. Zu diesem Zweck kann die Koppeleinheit entsprechend ausgebildet sein. Auch das Koppelmodul des Zubehörteils ist vorzugsweise entsprechend angepasst ausgebildet, sodass eine Koppelanordnung erreicht werden kann, die dem Übertragen von Daten zu dienen vermag.

Gemäß einer vorteilhaften Weiterbildung wird ferner vorgeschlagen, dass der Objekttisch eine Steuereinheit aufweist, die ausgebildet ist, eine Verbindung mit dem wenigstens einen Zubehörteil zu detektieren. Dadurch ist es möglich festzustellen, ob ein Zubehörteil am Objekttisch angeordnet ist und somit kurzfristig für die Nutzung zur Verfügung steht. Darüber hinaus kann vorgesehen sein, dass die Steuereinheit auch einen Betriebszustand des Zubehörteils erfasst. Beispielsweise kann erfasst werden, ob das Zubehörteil für einen drahtlosen Betrieb ausreichend mit elektrischer Energie versorgt ist. Es kann auch vorgesehen sein, dass die Steuereinheit erfasst, welche Parameter für den bestimmungsgemäßen Betrieb im Zubehörteil gespeichert sind. Auch eine Programmversion von einem Rechnerprogramm zum Betreiben einer Recheneinheit des Zubehörteils kann beispielsweise abgefragt werden. Die Steuereinheit kann diese Daten für eine Ausgabe an einen Nutzer oder auch für die weitere Unterstützung der Nutzung des bildgebenden Untersuchungsgeräts zur Verfügung stellen. Beispielsweise kann vorgesehen sein, dass Anzahl, Art, Betriebszustand und/oder dergleichen an eine zentrale Stelle übermittelt werden.

Es wird ferner vorgeschlagen, dass die Koppeleinheit wenigstens zwei elektrische Leiter zum leitungsgebundenen Koppeln des Zubehörteils aufweist, die sich zumindest teilweise parallel zum Tischrand erstrecken. Auf diese Weise kann erreicht werden, dass entlang der Erstreckung der Koppeleinheit eine Verbindung mit dem Zubehörteil hergestellt werden kann, die einen kontinuierlichen Koppelbereich zum Realisieren der Koppelanordnung bereitstellt. Vorzugsweise ist es über die Erstreckung der elektrischen Leiter möglich, an beispielsweise nahezu einer beliebigen Position eine Koppelanordnung mit dem Zubehörteil herzustellen. Die elektrischen Leiter können durch die Verbindungseinheit ausgebildet sein oder in diese integriert sein.

Gemäß einer Weiterbildung wird vorgeschlagen, dass die Koppeleinheit wenigstens ein Feldkopplungselement zum drahtlosen Koppeln des Zubehörteils aufweist. Mit dem Feldkopplungselement kann unter Nutzung eines Feldes eine drahtlose Übertragung von Energie und/oder Daten erreicht werden. Das Feld kann zum Beispiel ein Magnetfeld, ein elektrisches Feld, Licht und/oder dergleichen sein. Das Feldkopplungselement ist vorzugsweise entlang einer Erstreckung der Koppeleinheit beziehungsweise der Verbindungseinheit ausgebildet. Das Feldkopplungselement kann zum drahtlosen Koppeln von wenigstens einem Zubehörteil ausgebildet sein. Es kann aber auch vorgesehen sein, dass das Feldkopplungselement zum drahtlosen Koppeln von genau einem Zubehörteil ausgebildet ist. Es ist auch möglich, dass das Feldkopplungselement zum drahtlosen Koppeln von mehreren Zubehörteilen ausgebildet ist.

Es kann ferner vorgesehen sein, dass die Koppeleinheit mehrere Feldkopplungselemente aufweist, die an vorgebbaren Positionen der Verbindungseinheit angeordnet sind. Dabei ist vorzugsweise in jeweiliges Feldkopplungselement zum drahtlosen Koppeln von genau einem Zubehörteil ausgebildet. Die Feldkopplungselemente können im Wesentlichen gleich ausgebildet sein. Es kann aber auch vorgesehen sein, dass die Feldkopplungselemente spezifisch für ein jeweiliges Zubehörteil angepasst ausgebildet sind. Beispielsweise kann vorgesehen sein, dass ein Feldkopplungselement lediglich zum Übertragen von Energie ausgebildet ist, wohingegen ein anderes Feldkopplungselement lediglich zum Übertragen von Daten ausgebildet ist. Eine Übertragung von Daten kann beispielsweise unidirektional oder auch bidirektional vorgesehen sein. Auch eine Kombination hiervon kann vorgesehen sein.

Es wird vorgeschlagen, dass das wenigstens eine Feldkopplungselement zumindest zum induktiven Koppeln oder zumindest zum kapazitiven Koppeln ausgebildet ist. Insbesondere kann vorgesehen sein, dass das wenigstens eine Feldkopplungselement zum induktiven Koppeln ausgebildet ist. Dadurch ist es möglich eine Koppelanordnung unter Nutzung eines magnetischen Feldes zu realisieren. Das magnetische Feld ist vorzugsweise ein magnetisches Wechselfeld. Das Feldkopplungselement kann auf diese Weise nicht nur elektrische Energie, sondern auch Daten übertragen. Je nach Bedarf kann die Kopplung entweder unidirektional oder bidirektional ausgebildet sein.

Darüber hinaus wird vorgeschlagen, dass das wenigstens eine Feldkopplungselement zum kapazitiven Koppeln ausgebildet ist. Das Feldkopplungselement kann - ebenso wie das zum induktiven Koppeln ausgebildete Feldkopplungselement - unter Nutzung eines elektrischen Feldes, insbesondere eines elektrischen Wechselfeldes, ein Koppeln realisieren. Auch hier ist sowohl eine Übertragung von Energie als auch von Daten möglich. Auch eine Kombination hiervon ist realisierbar.

Ferner wird vorgeschlagen, dass das wenigstens eine Feldkopplungselement eine planare beziehungsweise flache ebene Struktur aufweist. Die Struktur kann auf einem Substrat wie beispielsweise einer Leiterplatte oder dergleichen angeordnet sein. Vorzugsweise ist die Struktur durch ein elektrisch leitfähiges Material wie Kupfer, Aluminium, Silber, Legierungen hiervon oder dergleichen gebildet. Die Struktur kann zum Beispiel spiralförmig, insbesondere nach Art einer archimedischen Spule, schneckenförmig und/oder mäanderförmig oder dergleichen ausgebildet sein. Hierbei können bedarfsweise auch mehrere Lagen vorgesehen sein, beispielsweise unter Nutzung mehrerer übereinander benachbart angeordneter Leiterplatten, um zum Beispiel bei einem als Spule ausgebildeten induktiven Feldkopplungselement eine möglichst große Anzahl von Windungen pro Flächeneinheit erreichen zu können.

Sowohl für das induktive Koppeln als auch für das kapazitive Koppeln weist die Koppeleinheit geeignete Einrichtungen auf, die das jeweilige Feldkopplungselement in entsprechender Weise zu betreiben vermögen. Das Zubehörteil weist jeweils ein entsprechendes Gegenelement auf, sodass ein induktives Koppeln beziehungsweise ein kapazitives Koppeln realisiert werden kann.

Gemäß einer Weiterbildung ist die Steuereinheit ausgebildet, das wenigstens eine Kopplungselement zu betreiben, wobei die Steuereinheit insbesondere ausgebildet ist, das wenigstens eine Feldkopplungselement in einem vom bildgebenden Untersuchungsgerät ungenutzten Frequenzbereich zu betreiben. Diese Weiterbildung basiert auf dem Gedanken, dass das Feldkopplungselement ein Wechselfeld nutzt, zum Beispiel in Bezug auf das induktive Koppeln ein magnetisches Wechselfeld sowie in Bezug auf das kapazitive Koppeln ein elektrisches Wechselfeld. Zu diesem Zweck kann die Steuereinheit geeignete elektronische Einrichtungen wie einen Wechselrichter und/oder dergleichen aufweisen. Darüber hinaus kann eine Codiereinheit vorgesehen sein, die es ermöglicht, nicht nur Energie, sondern auch Daten zu übertragen.

Vorzugsweise betreibt die Steuereinheit das wenigstens eine Feldkopplungselement in einem vom bildgebenden Untersuchungsgerät ungenutzten Frequenzbereich. Dadurch kann eine spektrale Entkopplung der Energieübertragung und/oder der Datenübertragung vom bestimmungsgemäßen Betrieb des bildgebenden Untersuchungsgeräts erreicht werden. Es ist also möglich, die Koppelanordnung zu realisieren und zu betreiben, und zwar im Wesentlichen unabhängig vom Betrieb des bildgebenden Untersuchungsgeräts.

Darüber hinaus wird vorgeschlagen, dass die Koppeleinheit mehrere Feldkopplungselemente aufweist, die an dem Tischrand entlang einer Erstreckungsrichtung der Verbindungseinheit nebeneinander angeordnet sind, wobei eine Erstreckungsrichtung des Tischrands mit der Erstreckungsrichtung der Verbindungseinheit zusammenfällt. Dadurch ist es möglich, ein jeweiliges Zubehörteil nahezu an einer beliebigen Position des Tischrands anzuordnen. Das Zubehörteil kann beispielsweise an einer für die Bedienung und die Durchführung der Untersuchung günstigen Position angeordnet werden. Die Feldkopplungselemente können in einem vorgegebenen Abstand beabstandet voneinander angeordnet sein. Die Erstreckungsrichtung der Verbindungseinheit ist vorzugsweise an die Erstreckungsrichtung des Tischrandes angepasst, sodass die beiden Erstreckungsrichtungen zusammenfallen können. Die Erstreckungsrichtung braucht jedoch keine Gerade darzustellen. Die Erstreckungsrichtung kann auch eine oder mehrere Krümmungen, Ecken und/oder dergleichen aufweisen.

Vorzugsweise ist entlang der Erstreckungsrichtung im Bereich von wenigstens einem jeweiligen Feldkopplungselement ein Rastelement angeordnet. Mittels des Rastelements kann erreicht werden, dass eine oder mehrere vorgegebene Positionen zum Verbinden eines jeweiligen Zubehörteils mit der Verbindungseinheit bestimmt werden können. Beispielsweise kann vorgesehen sein, dass ein Rastelement im Bereich eines jeweiligen Feldkopplungselements ausgebildet beziehungsweise angeordnet ist. Ist beispielsweise die Verbindungseinheit durch eine entsprechend ausgebildete Verbindungsnut gebildet, können die Rastelemente so ausgebildet sein, dass beim Verschieben des Verbindungselements des Zubehörteils in der Nut ein jeweiliges Rastelement eine taktile Rückmeldung ermöglicht, sodass einem Nutzer angezeigt werden kann, an welcher Stelle ein jeweiliges Feldkopplungselement zum Ausbilden der Koppelanordnung vorhanden ist. Es braucht also keine Sichtverbindung vorzugliegen.

Gemäß einer Weiterbildung wird vorgeschlagen, dass die Steuereinheit ausgebildet ist, die Feldkopplungselemente im Zeitmultiplex zu betreiben. Dem Grunde nach besteht natürlich die Möglichkeit, die Feldkopplungselemente gemeinsam, insbesondere zeitgleich zu betreiben. Vorteilhaft ist es jedoch, wenn die Feldkopplungselemente im Zeitmultiplex betrieben werden können, wodurch Aufwand seitens der Steuereinheit in Bezug auf die Energiebereitstellung oder eine Kapazität in Bezug auf die Kommunikation begrenzt werden kann.

Darüber hinaus wird vorgeschlagen, dass der Objekttisch eine Desinfektionseinheit aufweist, die ausgebildet ist, das wenigstens eine mit der Verbindungseinheit verbundene Zubehörteil zumindest teilweise zu desinfizieren. Diese Weiterbildung eignet sich insbesondere für den Fall, dass das Zubehörteil mit mehreren unterschiedlichen Objekten, die untersucht werden sollen, in Kontakt kommen kann. Dadurch kann vermieden werden, dass Kontaminationen, insbesondere Keime, von einem Objekt auf ein anderes Objekt übertragen werden können. Besonders vorteilhaft gilt dies natürlich dann, wenn das Objekt ein menschlicher oder tierischer Körper ist, der hinsichtlich seiner Struktur untersucht werden soll.

In Bezug auf das bildgebende Untersuchungsgerät wird ferner vorgeschlagen, dass das bildgebende Untersuchungsgerät ausgebildet ist, eine Verbindung von wenigstens einem Zubehörteil mit dem Objekttisch zu erfassen. Dadurch kann erreicht werden, dass untersuchungsgerätseitig eine Information dazu vorliegt, ob ein Zubehörteil am Objekttisch angeordnet und mit diesem verbunden ist. Diese Information kann weiterverarbeitet werden, beispielsweise indem entsprechende Daten bereitgestellt werden, die einer weiteren Datenverarbeitung zugeführt werden können. Es kann auch vorgesehen sein, dass im Bereich des bildgebenden Untersuchungsgeräts angezeigt werden kann, welche Zubehörteile mit dem Objekttisch verbunden sind. Dies ist vorteilhaft zur Vorbereitung einer Untersuchung, bei der - abhängig vom zu untersuchenden Objekt, dessen Zustand und/oder dergleichen - ein jeweiliges Zubehörteil zumindest zweitweise während der Untersuchung genutzt werden können soll. Insgesamt kann eine weitere Verbesserung erreicht werden.

Die vorstehend in der Beschreibung genannten Merkmale und Merkmalskombinationen sowie die nachfolgend in der Figurenbeschreibung genannten und/oder in den Figuren alleine gezeigten Merkmale und Merkmalskombinationen sind nicht nur in der jeweils angegebenen Kombination, sondern auch in anderen Kombinationen verwendbar, ohne den Rahmen der Erfindung zu verlassen.

Bei den im Folgenden erläuterten Ausführungsbeispielen handelt es sich um bevorzugte Ausführungsformen der Erfindung. Die vorhergehend in der Beschreibung angegebenen Merkmale, Merkmalskombinationen sowie auch die in der folgenden Beschreibung von Ausführungsbeispielen genannten und/oder in den Figuren allein gezeigten Merkmale und Merkmalskombinationen sind nicht nur in der jeweils angegebenen Kombination, sondern auch in anderen Kombinationen verwendbar. Es sind somit auch Ausführungen von der Erfindung umfasst beziehungsweise als offenbart anzusehen, die in den Figuren nicht explizit gezeigt und erläutert sind, jedoch durch separierte Merkmalskombinationen aus den erläuterten Ausführungsformen hervorgehen und erzeugbar sind. Die anhand der Ausführungsbeispiele dargestellten Merkmale, Funktionen und/oder Wirkungen können für sich genommen jeweils einzelne, unabhängig voneinander zu betrachtende Merkmale, Funktionen und/oder Wirkungen der Erfindung darstellen, welche die Erfindung jeweils auch unabhängig voneinander weiterbilden. Daher sollen die Ausführungsbeispiele auch andere Kombinationen als die in den erläuterten Ausführungsformen umfassen. Darüber hinaus können die beschriebenen Ausführungsformen auch durch weitere der bereits beschriebenen Merkmale, Funktionen und/oder Wirkungen der Erfindung ergänzt sein.

In den Figuren bezeichnen gleiche Bezugszeichen gleiche Merkmale beziehungsweise Funktionen.

Unabhängig vom grammatikalischen Geschlecht eines bestimmten Begriffes sind Personen mit männlicher, weiblicher oder anderer Geschlechteridentität mit umfasst.

Es zeigen:
FIG 1 eine Magnetresonanzuntersuchungsanordnung mit einem Magnetresonanzgerät und einer mit dem Magnetresonanzgerät verbundenen MR-Lokalspule, die an einem Patienten zum Durchführen einer Untersuchung am Patienten angeordnet ist;
FIG 2 eine perspektivische Ansicht des Magnetresonanzgeräts gemäß FIG 1 mit einem an einem Rand einer Mündung einer Durchgangsöffnung des Magnetresonanzgeräts angeordneten Objekttisch;
FIG 3 einen Ausschnitt aus einer schematisch perspektivischen Darstellung einer Unterseite eines Tischrands des Objekttisches gemäß FIG 2;
FIG 4 eine schematische Schnittdarstellung im Bereich des Tischrands; und
FIG 5 in einer schematischen Seitenansicht eine weitere Ausgestaltung für einen Objekttisch.

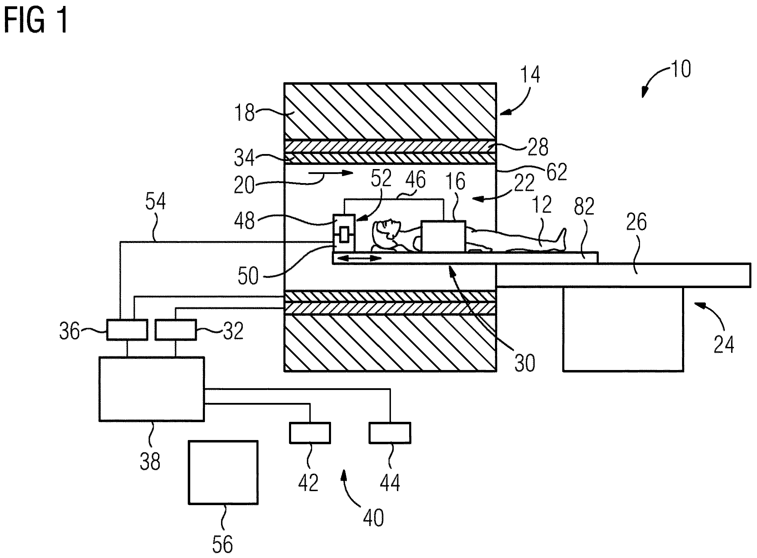

FIG 1 zeigt in einer teilweise geschnittenen Darstellung eine Magnetresonanzuntersuchungsanordnung 10, die ein Magnetresonanzgerät 14 als bildgebendes Untersuchungsgerät und eine Lokalspule 16 aufweist. Die Magnetresonanzuntersuchungsanordnung 10 dient vorliegend zur Durchführung eines bildgebenden Verfahrens der medizinischen Untersuchung eines Patienten 12 als zu untersuchendem Objekt. Die Magnetresonanzuntersuchungsanordnung 10 ist jedoch nicht auf diese Anwendung beschränkt und kann bei einer Vielzahl von anderen Untersuchungen an Objekten eingesetzt werden.

Das Magnetresonanzgerät 14 weist einen Hauptmagneten 18 auf, der dem Erzeugen eines starken, insbesondere zeitlich konstanten, Hauptmagnetfelds in einer Richtung 20 dient. Das Magnetresonanzgerät 14 weist ferner eine Durchgangsöffnung 22 als Erfassungsbereich auf, die vorliegend als Tunnel bereitgestellt ist und die einer Aufnahme des Patienten 12 dient. Die Durchgangsöffnung 22 ist vorliegend im Wesentlichen zylinderförmig ausgebildet und in einer Umfangsrichtung vom Hauptmagneten 18 umgeben. Grundsätzlich kann die Durchgangsöffnung 22 jedoch auch abweichend hiervon ausgebildet sein.

Das Magnetresonanzgerät 14 weist ferner eine Patientenlagerungsvorrichtung 24 beziehungsweise eine Objektlagerungsvorrichtung auf, die es ermöglicht, den Patienten 12 in die Durchgangsöffnung 22 zu bewegen. Zu diesem Zweck weist die Patientenlagerungsvorrichtung 24 einen zumindest teilweise innerhalb der Durchgangsöffnung 22 bewegbar geordneten Patiententisch 26 als Objekttisch auf. Die Erfindung ist jedoch auf diese Konstruktion des Erfassungsbereichs nicht beschränkt.

Das Magnetresonanzgerät 14 weist ferner eine Gradientenspuleneinheit 28 auf, mittels der Magnetfeldgradienten erzeugt werden können, die für eine Ortskodierung während einer bildgebenden Untersuchung verwendet werden können. Die Gradientenspuleneinheit 28 wird vorliegend mittels einer Gradientensteuereinheit 32 des Magnetresonanzgeräts 14 gesteuert. Der Hauptmagnet 18 sowie die Gradientenspuleneinheit 28 bilden eine Magnetfeldquelle.

Das Magnetresonanzgerät 14 weist ferner eine Hochfrequenzantenneneinheit 34 auf, die in der vorliegenden Ausgestaltung in das Magnetresonanzgerät 14 als Körperspule fest integriert ausgebildet ist. Die Hochfrequenzantenneneinheit 34 wird mittels einer Hochfrequenzantennensteuereinheit 36 des Magnetresonanzgeräts 14 gesteuert. Hiermit ist es möglich, hochfrequente Magnetresonanzsequenzen im Untersuchungsbereich 30 zu erzeugen. Im bestimmungsgemäßen Betrieb des Magnetresonanzgeräts 14 kann eine Anregung von Atomkernen im Untersuchungsbereich 30 beim Patienten 12 erreicht werden. Durch Relaxation der angeregten Atomkerne werden MR-Signale erzeugt. Mittels der Hochfrequenzantenneneinheit 34 können die MR-Signale empfangen werden.

Zur Steuerung der Komponenten des Magnetresonanzgeräts 14 weist das Magnetresonanzgerät 14 eine Gerätsteuereinheit 38 auf. Die Gerätsteuereinheit 38 steuert die Magnetresonanzuntersuchungsanordnung 10 zentral, beispielsweise in Bezug auf das Durchführen einer vorbestimmten Untersuchung am Patienten 12, insbesondere das Durchführen einer vorbestimmten bildgebenden Gradientenechosequenz. Darüber hinaus weist die Gerätsteuereinheit 38 eine nicht weiter dargestellte Auswerteeinheit auf, mittels der empfangene MR-Signale ausgewertet werden können, die während der Untersuchung erfasst werden.

Das Magnetresonanzgerät 14 weist ferner eine Benutzerschnittstelle 40 auf, die mit der Steuereinheit 38 kommunikationstechnisch verbunden ist. Die Benutzerschnittstelle 40 weist eine Anzeigeeinheit 42 sowie eine Eingabeeinheit 44 auf, die jeweils kommunikationstechnisch mit der Gerätsteuereinheit 38 verbunden sind. Über die Anzeigeeinheit 42 können visuell Daten und Informationen, Auswerteergebnisse, beispielsweise Bildgebungsergebnisse, Parameter in Bezug auf die Auswertung und/oder dergleichen dargestellt werden. Die Anzeigeeinheit 42 kann beispielsweise einen Monitor oder dergleichen aufweisen. Mittels der Benutzerschnittstelle 40 ist es für einen Nutzer des Magnetresonanzgeräts 14 sowie der Magnetresonanzuntersuchungsanordnung 10 möglich, der Gerätsteuereinheit 38 Daten zuzuführen, beispielsweise Parameter für die Durchführung der Untersuchung, Steuerdaten, Informationen und/oder dergleichen.

Aus FIG 1 ist ersichtlich, dass am Patienten 12 im Untersuchungsbereich 30 eine Lokalspule 16 angeordnet ist. Die Lokalspule 16 ist vorliegend an einer Oberfläche des Patienten 12 im Untersuchungsbereich 30 angeordnet. Die Lokalspule 16 weist ein Verbindungskabel 46 auf, welches in einem Verbindungsbereich 52 mit einem Anschlusskabel 54 des Magnetresonanzgeräts 14 kommunikationstechnisch und/oder signaltechnisch verbunden ist. Im Verbindungsbereich 52 ist zu diesem Zweck ein Spulenverbindungselement 48 vorgesehen, welches vorliegend mit dem Verbindungskabel 46 fest verbunden ist. Das Spulenverbindungselement 48 ist vorliegend als Stecker ausgebildet.

Im Verbindungsbereich 52 ist ferner ein Geräteverbindungselement 50 vorgesehen, welches vorliegend mit dem Anschlusskabel 54 fest verbunden ist. Mit dem Spulenverbindungselement 48 und dem Geräteverbindungselement 50 kann im Verbindungsbereich 52 eine lösbare Verbindung der Lokalspule 16 mit dem Magnetresonanzgerät 14 realisiert werden. Auf diese Weise ist es möglich, eine jeweilige Lokalspule 16 mit dem jeweiligen Magnetresonanzgerät 14 zu koppeln. Das Geräteverbindungselement 50 kann als Steckdose ausgebildet sein. Der Verbindungsbereich 52 ist vorliegend ein standardisierter Verbindungsbereich, sodass eine Verbindung von beliebigen Lokalspulen 16 mit beliebigen Magnetresonanzgeräten 14 basierend auf diesem Standard erreicht werden kann. Sowohl das Spulenverbindungselement 48 als auch das Geräteverbindungselement 50 weisen vorliegend eine Schirmung auf.

In FIG 1 ist lediglich eine einzige Verbindungseinheit 52 zum Anschließen einer einzigen Lokalspule 16 vorgesehen. In alternativen Ausgestaltungen kann natürlich vorgesehen sein, dass mehrere Verbindungseinheiten 52 vorgesehen sind, die es erlauben, mehr als eine einzige Lokalspule anzuschließen. Darüber hinaus kann vorgesehen sein, dass das Geräteverbindungselement 50 an einer beliebigen geeigneten Stelle des Patiententisches 26 angeordnet sein kann. Es kann auch vorgesehen sein, dass mehr als ein einziges Geräteverbindungselement 50 vorgesehen ist, wobei vorzugsweise die mehreren Geräteverbindungselemente 50 an unterschiedlichen Positionen, insbesondere des Patiententisches 26 angeordnet sein können. Die Hochfrequenzantenneneinheit 34 kann bedarfsweise entsprechend angepasst ausgebildet sein.

Die Magnetresonanzuntersuchungsanordnung 10 weist ferner eine Energieversorgungseinrichtung 56. Die Energieversorgungseinrichtung 56 ist vorliegend an ein dreiphasiges Energieversorgungsnetz 58 angeschlossen.

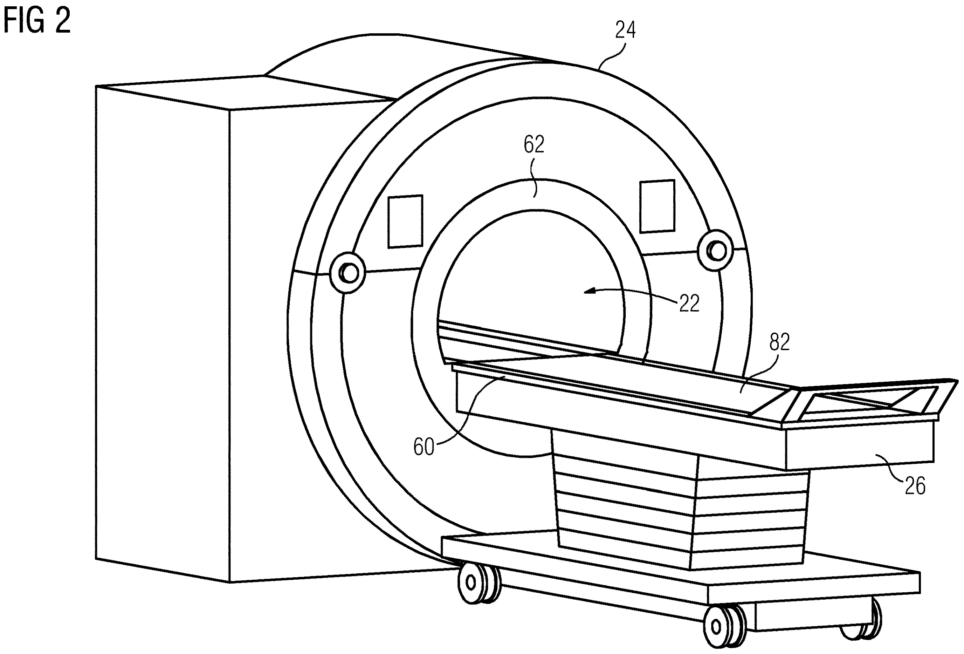

FIG 2 zeigt eine perspektivische Ansicht des Magnetresonanzgeräts 14 mit einem an einem Rand einer Mündung 62 der Durchgangsöffnung 22 des Magnetresonanzgeräts 14 angeordneten Objekttisch beziehungsweise Patiententisch 26. Der Objekttisch beziehungsweise Patiententisch 26 ist vorliegend bewegbar ausgebildet und weist zu diesem Zweck nicht bezeichnete Rollen auf. Der Objekttisch 26 kann bedarfsweise am Magnetresonanzgerät 14 angeordnet werden.

Der Objekttisch beziehungsweise Patiententisch 26 weist eine Objektauflage beziehungsweise Patientenauflage 82 auf, auf der der Patient 12 angeordnet werden kann. Die Patientenauflage 82 ist in Längsrichtung des Patiententisches 26 verfahrbar ausgebildet, sodass ein auf der Patientenauflage 82 angeordneter Patient 12 mittels der Patientenauflage 82 bedarfsweise in der Durchgangsöffnung 22 angeordnet werden kann.

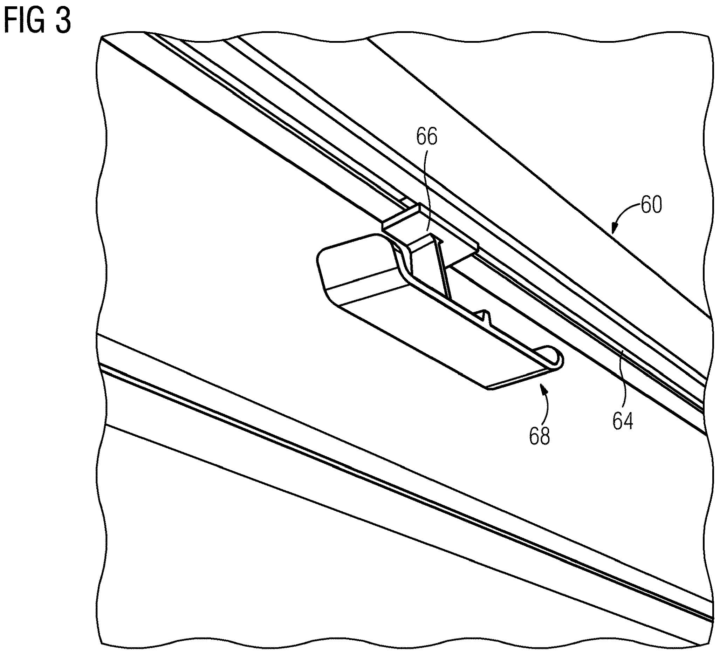

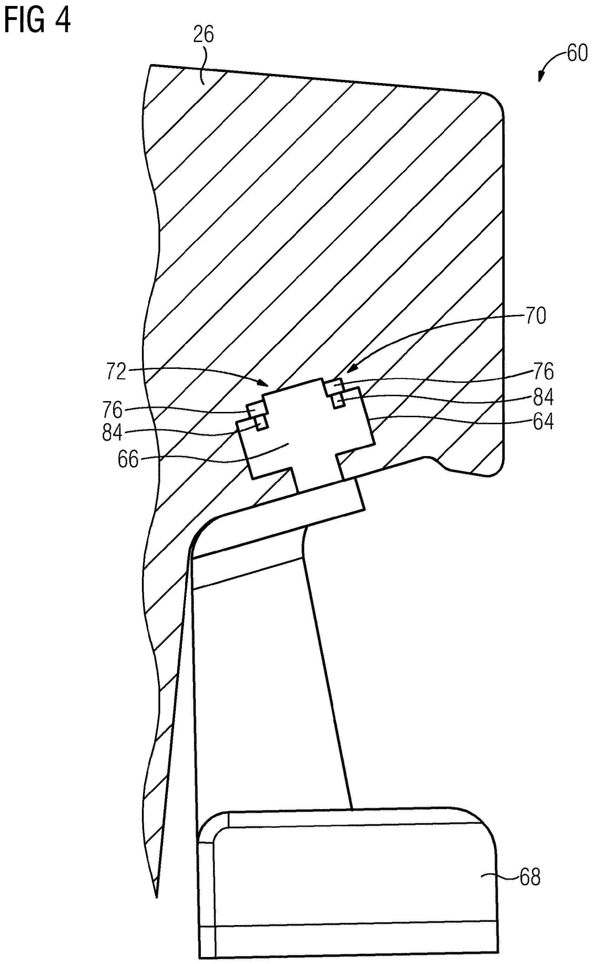

Aus FIG 2 ist ferner ersichtlich, dass der Objekttisch 26 einen Tischrand 60 aufweist. Der Tischrand 60 ist in den FIG 3 und 4 gemäß einer ersten Ausgestaltung der Erfindung vergrößert dargestellt.

FIG 3 zeigt einen Ausschnitt aus einer schematisch perspektivischen Darstellung einer Unterseite des Tischrands 60, wohingegen FIG 4 eine Schnittdarstellung im Bereich des Tischrands 60 darstellt. Aus den FIG 3 und 4 ist ersichtlich, dass entlang des Tischrands 60 eine als T-Nut ausgebildete Verbindungseinheit 64 ausgebildet ist. Die Verbindungseinheit 64 ist ausgebildet, mit einem Verbindungselement 66 eines für die Untersuchung verwendbaren Zubehörteils, vorliegend ein Elektrokardiograph (EKG) 68, lösbar verbunden zu werden. Dadurch kann das Zubehörteil am Objekttisch 26 angeordnet und gelagert werden. Das EKG 68 ist vorliegend für einen drahtlosen Betrieb ausgelegt und weist für seinen bestimmungsgemäßen Betrieb einen nichtdargestellten elektrischen Energiespeicher in Form eines Akkumulators auf.

Die Verbindungseinheit 64 weist vorliegend eine Koppeleinheit 70 auf, die ihrerseits zwei elektrische Leiter 76 aufweist. Die elektrischen Leiter 76 können zum Beispiel als Metallschienen, insbesondere als Kupferschienen, ausgebildet sein. Die elektrischen Leiter 76 sind an eine nicht dargestellte elektrische Energiequelle angeschlossen, die vorliegend elektrische Energie in Form einer Kleinspannung, beispielsweise etwa 24 V Gleichspannung, bereitstellt. Die elektrische Energiequelle kann zum Beispiel mit der Energieversorgungseinrichtung 56 elektrisch gekoppelt sein und von dieser elektrische Energie beziehen.

Die Verbindungseinheit 64 ist ausgebildet, mit einem Verbindungselement 66 des EKG 68 lösbar verbunden zu werden. Das Verbindungselement 66 weist elektrische Kontaktstifte 84 auf, die im verbundenen Zustand die elektrischen Leiter 76 kontaktieren. Dadurch kann eine Koppelanordnung 72 bereitgestellt werden, mittels der das EKG 68 im verbundenen Zustand mit elektrischer Energie zum Aufladen des Akkumulators versorgt werden kann.

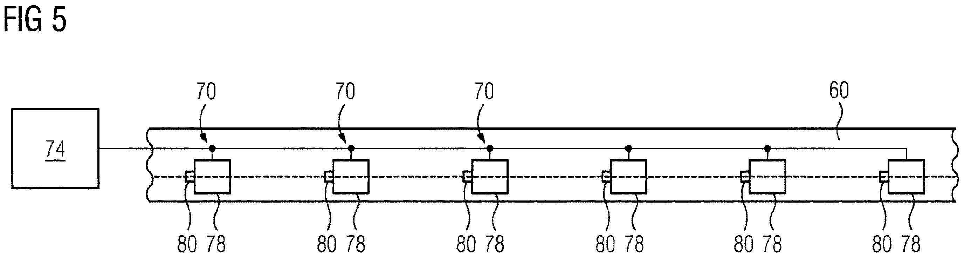

In alternativen Ausgestaltungen kann vorgesehen sein, dass die Koppelanordnung 72 nicht nur zum Übertragen von elektrischer Energie, sondern auch zum Übertragen von Daten genutzt werden kann. Das EKG 68 kann gemäß einer Ausgestaltung zur Triggerung bei einer Durchführung der Untersuchung am Objekt genutzt werden. In diesem Fall werden die Daten vorzugsweise instantan ausgewertet. Darüber hinaus können beispielsweise Steuerparameter oder Betriebsparameter, wie Temperatur, Ladungszustand des Energiespeichers und/oder Versionen von Betriebsprogrammen des EKG 68 übertragen werden. Darüber hinaus können alternativ oder ergänzend beispielsweise aus dem bestimmungsgemäßen Betrieb des EKG 68 angefallene Daten über die Koppelanordnung 72 an eine Steuereinheit 74 (FIG 5) übermittelt werden, mittels der diese Daten ausgewertet werden können. Diese Daten beziehungsweise deren Auswertung können dann durch die Steuereinheit 74 für eine weitere Verwendung bereitgestellt werden.

In einer weiteren Ausgestaltung kann vorgesehen sein, dass über die Koppelanordnung 72 gemäß der FIG 3 und 4 ausschließlich Daten übertragen werden.

FIG 5 zeigt in einer schematischen Seitenansicht eine weitere Ausgestaltung für einen Objekttisch beziehungsweise Patiententisch 26. Im Unterschied zu den vorhergehend beschriebenen Ausgestaltungen ist bei dieser Ausgestaltung vorgesehen, dass die Verbindungseinheit 64 Feldkopplungselemente 78 aufweist, die vorliegend zum induktiven Koppeln ausgebildet sind. In alternativen Ausgestaltungen kann hier auch vorgesehen sein, dass die Feldkopplungselemente 78 zum kapazitiven Koppeln ausgebildet sind.

Die Steuereinheit 74 ist ausgebildet, die Feldkopplungselemente 78 zu betreiben. Dabei betreibt die Steuereinheit 74 die Feldkopplungselemente 78 in einem vom Magnetresonanzgerät 14 ungenutzten Frequenzbereich. Dadurch kann eine spektrale Entkopplung der Nutzung des Magnetresonanzgeräts 14 vom Betrieb der Steuereinheit 74 und der Feldkopplungselemente 78 erreicht werden.

In der vorliegenden Ausgestaltung ist vorgesehen, dass die Feldkopplungselemente 78 als elektrische Spulen ausgebildet sind, die eine Spulenachse aufweisen, die vorliegend quer zur Mündung 62 ausgerichtet ist. Die elektrischen Spulen, die als Koppelspulen dienen können, können als planare beziehungsweise eben flache Struktur auf einer Leiterplatte ausgebildet sein. Die elektrischen Spulen können spiralförmig, zum Beispiel nach Art einer archimedischen Spule, oder schneckenförmig und/oder mäanderförmig ausgebildet sein. Hierbei können auch mehrere Lagen verwendet werden, beispielsweise unter Nutzung mehrerer Leiterplatten, um die Anzahl der Windungen einer jeweiligen Spule pro Flächeneinheit zu erhöhen. Darüber hinaus sind die Spulenachsen vorliegend im Wesentlichen horizontal ausgerichtet. Die Feldkopplungselemente 78 sind entlang einer Erstreckungsrichtung der Verbindungseinheit 64 nebeneinander angeordnet. Die Erstreckungsrichtung des Tischrands 60 fällt mit der Erstreckungsrichtung der Verbindungseinheit 64 zusammen.

Im Bereich eines jeweiligen Feldkopplungselements 78 ist ferner ein jeweiliges Rastelement 80 angeordnet. Dies ermöglicht es, insbesondere wenn die Verbindungseinheit 64 nach Art einer T-Nut ausgebildet ist, das jeweilige Verbindungselement 66 an einem jeweiligen Ende der T-Nut einzuführen und im Bereich eines jeweiligen Feldkopplungselements 78 anzuordnen. Durch das Rastelement 80 kann eine taktile Rückmeldung realisiert werden, die es ermöglicht, beim Durchschieben des Verbindungselements 66 durch die T-Nut eine Position eines jeweiligen Feldkopplungselements 78 taktil zu erfassen und dadurch die Koppelanordnung 72 drahtlos herzustellen.

Die Steuereinheit 74 ist ferner ausgebildet, zu erfassen, welches der Feldkopplungselemente 78 mit einem Zubehörteil wie dem EKG 68 gekoppelt ist. Nur die Feldkopplungselemente 78 werden betrieben, bei denen eine jeweilige Kopplungsanordnung 72 ausgebildet ist. Sind mehrere Zubehörteile mit jeweiligen Feldkopplungselementen 78 gekoppelt, kann die Steuereinheit 74 ausgebildet sein, die Feldkopplungselemente 78 im Zeitmultiplex zu betreiben.

Die Ausführungsbeispiele dienen ausschließlich der Erläuterung der Erfindung und sollen diese nicht beschränken.

## Patentansprüche

1. Objekttisch (26) für ein bildgebendes Untersuchungsgerät (14), das zum Durchführen einer Untersuchung an einem auf dem Objekttisch (26) anordbaren Objekt (12) dient, wobei der Objekttisch (26) ausgebildet ist, an einem Rand eines Erfassungsbereichs (22) des bildgebenden Untersuchungsgeräts (14) angeordnet zu werden, um das zu untersuchende Objekt (12) zum Durchführen der Untersuchung im Erfassungsbereich (22) anzuordnen, wobei der Objekttisch (26) an einem Tischrand (60) zumindest bereichsweise eine entlang des Tischrands (60) verlaufende Verbindungseinheit (64) aufweist, die ausgebildet ist, mit einem Verbindungselement (66) wenigstens eines für die Untersuchung verwendbaren Zubehörteils (68) lösbar verbunden zu werden,
**dadurch gekennzeichnet, dass**
die Verbindungseinheit (64) eine Koppeleinheit (70) aufweist, wobei die Koppeleinheit (70) ausgebildet ist, in einem verbundenen Zustand, in dem die Verbindungseinheit (64) mit dem Verbindungselement (66) verbunden ist, mit dem Zubehörteil (68) eine Koppelanordnung (72) auszubilden, wobei die Koppelanordnung (72) einem Übertragen von zumindest Daten oder Energie dient.

2. Objekttisch nach Anspruch 1, **dadurch gekennzeichnet, dass** die Koppeleinheit (70) als elektrische Koppeleinheit zum leitungsgebundenen oder drahtlosen elektrischen Koppeln des Zubehörteils (68) ausgebildet ist.

3. Objekttisch nach einem der vorhergehenden Ansprüche, **dadurch gekennzeichnet, dass** die Koppeleinheit (70) als kommunikationstechnische Koppeleinheit zum leitungsgebundenen oder drahtlosen kommunikationstechnischen Koppeln des Zubehörteils (68) ausgebildet ist.

4. Objekttisch nach einem der vorhergehenden Ansprüche, **gekennzeichnet durch** eine Steuereinheit (74), die ausgebildet ist, eine Verbindung mit dem wenigstens einen Zubehörteil (68) zu detektieren.

5. Objekttisch nach einem der vorhergehenden Ansprüche, **dadurch gekennzeichnet, dass** die Koppeleinheit (70) wenigstens zwei elektrische Leiter (76) zum leitungsgebundenen Koppeln des Zubehörteils (68) aufweist, die sich zumindest teilweise parallel zum Tischrand (60) erstrecken.

6. Objekttisch nach einem der vorhergehenden Ansprüche, **dadurch gekennzeichnet, dass** die Koppeleinheit (70) wenigstens ein Feldkopplungselement (78) zum drahtlosen Koppeln des Zubehörteils (68) aufweist.

7. Objekttisch nach Anspruch 6, **dadurch gekennzeichnet, dass** das wenigstens eine Feldkopplungselement (78) zumindest zum induktiven Koppeln oder zumindest zum kapazitiven Koppeln ausgebildet ist.

8. Objekttisch nach Anspruch 7, **dadurch gekennzeichnet, dass** das wenigstens eine Feldkopplungselement (78) eine planare beziehungsweise flache ebene Struktur aufweist.

9. Objekttisch nach einem der Ansprüche 6 bis 8, **dadurch gekennzeichnet, dass** die Steuereinheit (74) ausgebildet ist, das wenigstens eine Feldkopplungselement (78) zu betreiben, wobei die Steuereinheit (74) insbesondere ausgebildet ist, das wenigstens eine Feldkopplungselement (78) in einem vom bildgebenden Untersuchungsgerät (14) ungenutzten Frequenzbereich zu betreiben.

10. Objekttisch nach einem der Ansprüche 6 bis 9, **dadurch gekennzeichnet, dass** die Koppeleinheit (70) mehrere Feldkopplungselemente (78) aufweist, die an dem Tischrand (60) entlang einer Erstreckungsrichtung der Verbindungeinheit (64) nebeneinander angeordnet sind, wobei eine Erstreckungsrichtung des Tischrands (60) mit der Erstreckungsrichtung der Verbindungeinheit (64) zusammenfällt.

11. Objekttisch nach Anspruch 10, **dadurch gekennzeichnet, dass** entlang der Erstreckungsrichtung im Bereich eines jeweiligen Feldkopplungselements (78) ein Rastelement (80) angeordnet ist.

12. Objekttisch nach einem der Ansprüche 10 oder 11, **dadurch gekennzeichnet, dass** die Steuereinheit (74) ausgebildet ist, die Feldkopplungselemente (78) im Zeitmultiplex zu betreiben.

13. Objekttisch nach einem der vorhergehenden Ansprüche, **gekennzeichnet durch** eine Desinfektionseinheit, die ausgebildet ist, das wenigstens eine mit der Verbindungseinheit (64) verbundene Zubehörteil (68) zumindest teilweise zu desinfizieren.

14. Bildgebendes Untersuchungsgerät (14) zum Durchführen einer Untersuchung an einem Objekt (12), mit einem Objekttisch (26) zum Anordnen des Objekts (12) sowie wenigstens einem Zubehörteil (68),
**dadurch gekennzeichnet, dass**
der Objekttisch (26) nach einem der vorhergehenden Ansprüche ausgebildet ist.

15. Bildgebendes Untersuchungsgerät nach Anspruch 14, **dadurch gekennzeichnet, dass** das bildgebende Untersuchungsgerät (14) ausgebildet ist, eine Verbindung von wenigstens einem Zubehörteil (68) mit dem Objekttisch (26) zu erfassen.
